# EUROPÄISCHE PATENTSCHRIFT

(11) **EP 2 559 395 B1**
(45) Veröffentlichungstag und Bekanntmachung des Hinweises auf die Patenterteilung: **17.10.2018**
(21) Anmeldenummer: 12005475.4
(22) Anmeldetag: 27.07.2012
(51) Int. Cl.: A61C 19/02, B65D 45/24, E05B 63/24, E05B 65/52, A61L 2/26

(54) **VERSCHLUSS FÜR STERILCONTAINER**
CLOSURE FOR STERILE CONTAINER
FERMETURE POUR CONTENEUR STÉRILE

(30) Priorität: 17.08.2011 DE 202011104615 U
(43) Veröffentlichungstag der Anmeldung: 20.02.2013
(73) Patentinhaber: Innovations Medical GmbH, 78532 Tuttlingen (DE)
(72) Erfinder: Kreidler, Winfried, 78532 Tuttlingen (DE); Kreidler, Jochen, 78532 Tuttlingen (DE)

(56) Entgegenhaltungen:
- DE-A1- 19 755 532
- GB-A- 190 901 818
- US-A- 1 827 319
- US-A- 4 915 913

## Beschreibung

Die Erfindung betrifft einen Verschluss zum Verriegeln eines aus einem Behälterdeckel und einem kastenartigen Unterteil bestehenden Sterilcontainers, wobei der Behälterdeckel und das Unterteil zur Bildung eines geschlossenen Sterilraums abnehmbar und mittels umlaufend zwischen dem Behälterdeckel und Unterteil angeordneten Dichtungsmitteln luftdicht auf das Unterteil aufsetzbar und mit diesem verbindbar ist, wobei der Verschluss einen Spannhebel aufweist, welcher aus einer geschlossenen über dem Todpunkt liegenden Spannstellung um eine Schwenkachse schwenkbar in eine geöffnete Schwenkstellung bringbar ist und, dass eine um eine Drehachse drehbare Verschlussklappe vorgesehen ist, welche in ihrer Schließstellung mit dem Spannhebel formschlüssig in Zugverbindung steht.

Verschlüsse für Sterilcontainer sind schon seit Jahren bekannt und dienen dazu, den Behälterdeckel und das in der Regel kastenartig ausgebildete Unterteil des Sterilcontainers lösbar und dicht miteinander zu verbinden. Um einen möglichst festsitzenden Halt des Behälterdeckels auf dem unterteil zu erreichen, werden u.a. hebelartig ausgebildete Verschlüsse eingesetzt, welche aus mehreren Bauteilen bestehen, die im geschlossenen Zustand rastend miteinander in Eingriff gebracht werden können. Eine weitere Variante von Verschlüssen arbeitet nach dem "Kniehebel"-Prinzip, wobei solche Verschlüsse mit einem Kipphebel und einem Zugbügel nach Art eines Schnellspannverschlusses ausgebildet sein können.

Aus der DE 197 55 532 A1 ist ein Verschluss bekannt, welcher nach dem "Rastprinzip" arbeitet. In dieser Druckschrift wird des Weiteren allgemein auf solche Verschlüsse eingegangen. So wird ausgeführt, dass das Zusammenspannen des Behälterdeckels und des Unterteils durch spezielle Verschlüsse ermöglicht werden kann, die eine an einem Teil (Behälterdeckel oder Unterteil) eine schwenkbar gelagerte Verschlussklappe und am jeweils anderen Teil eine feststehend fixierte Rastnase aufweisen. Die Rastnase kann dabei elastisch verformbar ausgebildet sein, so dass ein Rastvorsprung an der Verschlussklappe bei deren Schwenkbewegung in eine Rastausnehmung der Rastnase einrasten kann. Dabei wird bei der Schwenkbewegung die Rastnase elastisch so verformt, dass der Rastvorsprung in die Rastausnehmung "eingleiten" kann. Die notwendige Spannkraft zum Verspannen des Behälterdeckels gegen das Unterteil wird bei einer solchen Ausgestaltung eines Verschlusses durch die elastische Nachgiebigkeit der Rastnase bestimmt.

Da sich bei solchen Konstruktionen gewisse Schwierigkeiten beim Ausgleichen von Fertigungstoleranzen und bei der Anpassung der notwendigen Spannkraft zwischen Behälterdeckel und Unterteil ergeben, wird beim Gegenstand der DE 197 55 532 A1 vorgeschlagen, den Rastvorsprung in der Klappe in eine Richtung elastisch verschiebbar auszubilden. Der Rastvorsprung wird in der Verschlussklappe elastisch verschiebbar ausgebildet. Entsprechend der Abmessungen der Verschlussklappe steht ein relativ großer Verschiebeweg zur Verfügung, der wesentlich größer ist, als der Verschiebeweg einer elastischen verformbaren Rastnase. Durch diesen "vergrößerten" Verschiebeweg wird eine Anpassung an Fertigungstoleranzen erleichtert. Des Weiteren ist es möglich, die Federkraft zu variieren, mit welcher der Rastvorsprung in Richtung auf den "Rückspruch" verschoben wird.

Wird der Rastvorsprung einstückig mit der Verschlussklappe ausgebildet, so wird die elastische Verschiebbarkeit dadurch erreicht, dass im Verbindungsbereich zwischen dem Rastvorsprung und der Verschlussklappe elastisch verformbare Verbindungsglieder angeordnet sind, welche beispielsweise als federförmige Stege oder dergleichen ausgebildet sein können. In einer bevorzugten Ausführungsform ist vorgesehen, dass der Rastvorsprung als von der Verschlussklappe separater Rastkörper ausgebildet ist, der in der Klappe in einer Führung verschiebbar gelagert ist. Diese Ausgestaltung soll den Vorteil aufweisen, besonders einfach herstellbar zu sein.

In der Praxis hat sich für einen derartigen Verschluss gezeigt, dass dieser aufgrund der rastenden Verbindung einem erhöhten Verschleiß unterliegt und auch, dass bei längerer Betriebsdauer die Federkräfte der verwendeten Federelemente erlahmen, so dass die Gefahr des Abhebens des Behälterdeckels vom Unterteil bei höheren Innendrücken gegeben ist.

Des Weiteren sind derartige, rastende Verschlüsse auch aus der US 4,331,257 A bekannt. Auch hier ist eines der rastend miteinander in Eingriff bringbaren Rastelemente federbelastet nachgiebig, beispielsweise am Unterteil des Sterilbehälters, angeordnet. Dieser kann durch einen klappbaren Riegel, welcher ebenfalls mit einer Rastnase versehen ist, rastend hintergriffen werden, so dass damit der Sterilcontainer geschlossen ist.

Aus der DE 721 588 C ist wiederum ein rastender Verschluss bekannt, welcher als "Schnappverschluss" ausgebildet ist. Bei dieser Konstruktion ist ein schwenkbarer Bügel vorgesehen, welcher einen bogenförmig ausgebildeten Bügelabschnitt aufweist. Dieser Bügelabschnitt ist mit einer Rastvertiefung, beispielsweise des Unterteils des Sterilcontainers, rastend in Eingriff bringbar.

Da solche Sterilcontainer bei aufgesetztem Behälterdeckel dicht ausgebildet sein müssen, sind bei den rastenden Varianten die elastisch nachgiebigen Bauteile äußerst stabil auszuführen, so dass die miteinander in Rastverbindung zu bringenden Bauteile einem äußerst hohen Verschleiß unterliegen. Sind die, die beiden Bauteile rastend miteinander in Verbindung haltenden Rastkräfte zu gering, so besteht die Gefahr, dass während des Sterilisationsprozesses der Behälterdeckel vom Unterteil abgehoben wird und somit die Dichtheit nicht mehr gewährleistet ist.

Des Weiteren sind aus dem Stand der Technik auch nach dem Kniehebelprinzip und dem Übertotpunktprinzip arbeitende Verschlüsse bekannt. Bei diesen Verschlüssen ist ein Spannhebel vorgesehen, welcher über eine entsprechende Lagerung, beispielsweise am Unterteil des Sterilcontainers, schwenkbar gelagert ist. In einem gewissen Abstand zur Lagerachse des Spannhebels ist am Spannhebel ein Spannbügel schwenkbar gelagert. Beim Öffnen des Spannhebels bewegt sich dieser Spannbügel von der Lagerachse des Spannhebels weg und kann somit manuell in einen Zughaken eingehängt werden, welcher wiederum festsitzend am Behälterdeckel angeordnet ist. Beim Schließvorgang wird dieser Zughaken durch den Spannbügel auf Grund seiner exzentrischen Lagerung zur Lagerachse des Spannhebels in Richtung dieser Lagerachse gezogen, so dass der Behälterdeckel gegen das Unterteil verspannbar ist. Im vollständig geschlossenen Zustand des Spannhebels liegt die Schwenkachse des Spannbügels nach dem "Übertotpunktprinzip" näher an der Behälterwand des Unterteils als die Lagerachse des Spannhebels, so dass ein selbständiges Öffnen des Spannhebels nicht möglich ist.

Solche "klassischen" Kniehebelverschlüsse sind in ihrer Handhabung etwas umständlich, da insbesondere zum Schließen stets der am Spannhebel frei bewegliche Spannbügel manuell mit dem Zughaken in Eingriff gebracht werden muss.

Weiter sind aus der US 4,915,913 A verschiedene Ausführungsvarianten von Behälter-Verschlüssen bekannt, welche nach dem Übertotpunktprinzip arbeiten. Bei einer dieser Varianten ist ein Spannhebel vorgesehen, welcher schwenkbar am Behältergehäuse angeordnet ist. Am Behälterdeckel ist eine Verschlussklappe drehbar angeordnet. Die Verschlussklappe weist an ihrem beweglichen Ende ein etwa halbzylindrisch zum Spannhebel hin abgebogen verlaufendes Endprofil auf. Der Spannhebel ist mit einem Durchbruch versehen, dessen im geschlossenen Zustand zur Verschlussklappe hin gerichtete, obere Begrenzungskante ein ebenfalls etwa halbzylindrisch ausgebildetes Eingriffsprofil aufweist. Im geschlossenen Zustand durchragt das Endprofil der Verschlussklappe den Durchbruch des Spannhebels und steht mit dem Eingriffsprofil des Spannhebels in Zugverbindung. In diesem geschlossenen Zustand befindet sich die Formschlussverbindung zwischen der Endkante der Verschlussklappe und dem Eingriffprofil des Spannhebels in einem geringeren Abstand zur Außenwand des Behältergehäuses als die Drehachse der Verschlussklappe und die Schwenkachse des Spannhebels, so dass - nach dem Übertotpunktprinzip - auf den Spannhebel ein Drehmoment wirkt, welche den Spannhebel und damit den Behälterverschluss in der geschlossenen Stellung hält. Zum Öffnen dieses Verschlusses ist vorgesehen, dass der Spannhebel manuell aus seiner geschlossenen Schwenkstellung ausgelenkt wird. Durch die dadurch bewirkte Schwenkbewegung des Spannhebels wird gleichzeitig über die formschlüssige Verbindung zwischen der Endprofil der Verschlussklappe und dem Eingriffprofil des Spannhebels eine Drehbewegung der Verschlussklappe bewirkt. Bei dieser Schwenkbewegung des Spannhebels bewegt sich der Spannhebel mit seinem Durchbruch entlang der Verschlussklappe, so dass diese bis etwa zur Hälfte ihrer gesamten Länge den Durchbruch durchragt. In dieser geöffneten Stellung des Spannhebels liegt die Verschlussklappe am Eingriffsprofil des Spannhebels an und befindet sich mit ihrem Endprofil unterhalb des Eingriffsprofils. Das Eingriffprofil und das Endprofil stehen in dieser Stellung der Verschlussklappe und des Spannhebels nicht mehr miteinander in Eingriff. Um den Behälterdeckel jedoch vom Behältergehäuse abheben zu können, muss die Verschlussklappe manuell aus dieser halb geöffneten Stellung in eine weiter geöffnete Stellung gebracht werden, wobei der Spannhebel gleichzeitig in seiner geöffneten Stellung gehalten werden muss. Die Stellbewegung der Verschlussklappe ist dabei durch den Durchbruch des Spannhebels begrenzt. Befindet sich die Verschlussklappe in Ihrer maximal geöffneten Stellung, so muss nun der Spannhebel wieder in seine geschlossenen Stellung gebracht werden, so dass die Verschlussklappe mit dem Durchbruch des Spannhebels außer Eingriff gelangt. Nun erst kann der Behälterdeckel vom Behältergehäuse abgenommen werden. Es ist erkennbar, dass auch bei diesem Verschluss die Handhabung äußerst kompliziert ist.

Weiterhin offenbart die GB 01818 AD 1909 ein Verschluss mit einem Spannhebel und einer Verschlussklappe, welche in ihrer Schließstellung mit dem Spannhebel in formschlüssiger Zugverbindung steht.

Demgemäß liegt der Erfindung die Aufgabe zugrunde, einen Verschluss für einen Sterilcontainer mit einem Behälterdeckel und einem kastenartigen Unterteil (Behältergehäuse) derart auszugestalten, dass insbesondere eine äußerst einfache Handhabung gewährleistet ist, wobei ein möglichst geringer Verschleiß sichergestellt sein soll.

Die Aufgabe wird erfindungsgemäß zusammen mit den Merkmalen des Oberbegriffes des Anspruches 1 dadurch gelöst, dass der Spannhebel über die formschlüssige Verbindung mit der Verschlussklappe während der Drehbewegung der Verschlussklappe aus ihrer Schließstellung in ihre geöffnete Drehstellung durch die Verschlussklappe in seine geöffnete Schwenkstellung bringbar ist und, dass die Verschlussklappe nach einer weiteren Drehbewegung in ihre vollständig geöffneten Endstellung mit dem Spannhebel außer Eingriff gelangt und, dass der Spannhebel in seiner geöffneten Schwenkstellung fixiert ist und, dass die Verschlussklappe beim Schließvorgang mit dem Spannhebel formschlüssig in Eingriff gelangt und der Spannhebel durch die Verschlussklappe in seine geschlossene Spannstellung verstellbar ist.

Durch die erfindungsgemäße Ausgestaltung wird ein Verschluss mit einem äußerst einfachen Aufbau sowie einer äußerst einfachen Handhabung zur Verfügung gestellt.

Dabei ist vorgesehen, dass die Verschlussklappe mit einem Spannhebel im geschlossenen Zustand unter Zugspannung in Eingriff steht. Dieser Spannhebel ist aus seiner geschlossenen Spannstellung heraus schwenkbar und in eine geöffnete Schwenkstellung bringbar. Aufgrund der formschlüssigen Verbindung zwischen der Verschlussklappe und dem Spannhebel im geschlossenen Zustand ist der Spannhebel durch die Verschlussklappe beim Öffnen der Verschlussklappe aus seiner geschlossenen Spannstellung in seine geöffnete Schwenkstellung verstellbar. Wird die Verschlussklappe weiter bis in ihre geöffnete Endstellung gebracht, so gelangt diese mit dem Spannhebel außer Eingriff. In dieser Stellung kann der Behälterdeckel vom kastenartigen Unterteil des Sterilcontainers abgehoben werden. In entsprechend umgekehrter Richtung, nach Aufsetzen des Behälterdeckels auf das Unterteil, wird die Verschlussklappe manuell aus ihrer geöffneten Endstellung in Schließrichtung gedreht und gelangt wiederum mit dem Spannhebel in Eingriff. Zu diesem Zweck ist der Spannhebel in seiner geöffneten Schwenkstellung fixiert. Die Fixierung kann beispielsweise durch eine Schenkelfeder erfolgen. Auch eine Reibschluss im Bereich der Schwenklagerung des Spannhebels oder auch eine Art Rastverbindung ist denkbar.

Durch die entstandene, formschlüssige Verbindung zwischen der Verschlussklappe und dem Spannhebel, wird während der weiteren Schließbewegung der Verschlussklappe gleichzeitig der Spannhebel aus seiner geöffneten Schwenklage in seine geschlossene Spannstellung bewegt. In dieser geschlossenen Spannstellung befindet sich der Spannhebel bezüglich seiner Schwenkachse über dem Totpunkt, so dass der Spannhebel und die Verschlussklappe in dieser geschlossenen Stellung zwangsläufig fixiert sind. Damit ist der erfindungsgemäße Verschluss in einfacher Weise mit einer Hand betätigbar, wobei dessen mit einander in Wirkverbindung bringbaren Bauteile automatisch bei der Drehbewegung des Verschlussklappe miteinander in Eingriff bringbar und wieder voneinander lösbar sind.

Aufgrund dieses formschlüssigen Eingriffs und lediglich einer "drehenden" Relativbewegung der Verschlussklappe relativ zum Spannhebel im Bereich der formschlüssigen Verbindung unterliegen die Verschlussklappe und der Spannhebel lediglich einem äußert geringer Verschleiß.

Des Weiteren sind durch die besondere Ausgestaltung des Verschlusses äußerst große Schließkräfte auf die in der Regel zwischen dem Behälterdeckel und dem Unterteil vorgesehene Dichtungsmittel aufbringbar, so dass insbesondere bei einem inneren Überdruck innerhalb des Sterilcontainers ein Abheben des Behälterdeckels vom Unterteil und somit ein Undichtwerden sicher vermieden wird. Da bei dieser erfindungsgemäßen Lösung keine federelastischen Elemente vorgesehen sind, besteht ebenfalls nicht die Gefahr, dass die Schließ- oder Spannkräfte mit zunehmender Betriebdauer abnehmen.

Weitere vorteilhafte Ausgestaltungen der Erfindung sind den weiteren Unteransprüchen entnehmbar.

So kann gemäß Anspruch 2 vorgesehen sein, dass der Spannhebel blockartig ausgebildet ist und zwei voneinander beabstandete Lagerschenkel aufweist, welche jeweils mit einer Lagerbohrung versehen sind. Über diese Lagerschenkel ist der Spannhebel an einem am Sterilcontainer befestigten Lagerbock schwenkbar gelagert. Durch diese Ausgestaltung wird eine hohe Stabilität des Spannhebels erreicht. Des Weiteren kann zwischen den beiden Lagerschenkein eine Schenkelfeder angeordnet werden, durch welche der Spannhebel in seiner geöffneten nicht mit der Verschlussklappe in Eingriff stehenden Schwenkstellung gehalten wird.

Weiter kann gemäß Anspruch 3 vorgesehen sein, dass der Spannhebel ausgehend von den Lagerschenkeln einen Spannblock bildet und, dass der Spannblock in seinem den Lagerschenkeln gegenüberliegenden Endbereich eine parallel zu seiner Schwenkachse verlaufende Vertiefung aufweist, mit welcher die Verschlussklappe in formschlüssig und beweglich in Eingriff bringbar ist. Durch diese Ausgestaltung sind die Verschlussklappe und der Spannhebel sicher miteinander in Eingriff bringbar.

Zur sicheren Funktionsweise trägt auch die Ausgestaltung nach Anspruch 4 bei. Danach ist vorgesehen, dass die Vertiefung "rückseitig" zum Sterilcontainer hin durch einen etwa radial zur Schwenkachse verlaufenden Anschlagsteg begrenzt ist, welcher mit der Verschlussklappe beim Schließvorgang in Wirkverbindung gelangt und, dass sich an die Vertiefung dem Anschlagsteg gegenüberliegend eine Eingriffsfläche anschließt. Der Anschlagsteg kann dabei nicht nur als Anschlag für die Verschlussklappe dienen sondern auch die geschlossenen Spannstellung des Spannhebels definieren, indem dieser Anschlag in der geschlossenen Spannstellung beispielsweise am Sterilcontainer oder einem außenseitig am Sterilcontainer befindlichen Bauteil anliegt.

Um die Verschlussklappe "bediensicher" mit dem Spannhebel in Eingriff bringen zu können und die Verschlussklappe wieder einfach vom Spannhebel lösen zu können, sind die Ausgestaltungen gemäß der Ansprüche 5 und 6 vorgesehen.

So kann gemäß Anspruch 5 vorgesehen sein, dass die Verschlussklappe über ein Lagerelement drehbar am Sterilcontainer gelagert ist und, dass die Verschlussklappe einen zentralen Durchbruch mit einer parallel zur Drehachse verlaufenden Eingriffsleiste aufweist, mit welcher die Verschlussklappe mit dem Spannhebel formschlüssig in Eingriff bringbar ist.

Hierzu kann gemäß Anspruch 6 weiter vorgesehen sein, dass der Spannhebel mit seinem Spannblock im geschlossenen Zustand des Verschlusses den Durchbruch der Verschlussklappe durchragt und, dass der Durchbruch eine Eingriffsleiste bildet, welche im geschlossenen Zustand des Verschlusses mit der Vertiefung des Spannblocks formschlüssig und relativ zu diesem drehbar in Eingriff steht.

Anhand der Zeichnung wird beispielhaft das Funktionsprinzip des erfindungsgemäßen Verschlusses näher erläutert. Die dargestellten Bauteile sind hier lediglich beispielhaft und können in ihrer konstruktiven Formgebung auch anders ausgebildet sein. Es zeigt:
- Fig. 1: eine perspektivische Explosionsdarstellung einer möglichen Ausführungsvariante eines erfindungsgemäßen Verschlusses;
- Fig. 2: eine perspektivische Darstellung der Verschlussklappe in ihrem, in einem Lagerelement drehbar aufgenommenen Zustand;
- Fig. 3: eine perspektivische Darstellung des Spannhebels in seinem, an einem Lagerbock schwenkbar gelagerten Zustand;
- Fig. 4: einen perspektivischen Teilschnitt eines Sterilcontainers mit Behälterdeckel und kastenartigem Unterteil mit einem erfindungsgemäßen Verschluss in dessen geschlossenem Zustand;
- Fig. 5: eine Schnittdarstellung des Sterilcontainers aus Fig. 4 mit dem erfindungsgemäßen Verschluss in geschlossenem Zustand;
- Fig. 6: die Darstellung aus Fig. 5 mit dem Verschluss in seiner teilweise geöffneten Betriebsstellung;
- Fig. 7: die Darstellung aus Fig. 6 mit dem Verschluss in seiner geöffneten Betriebsstellung.

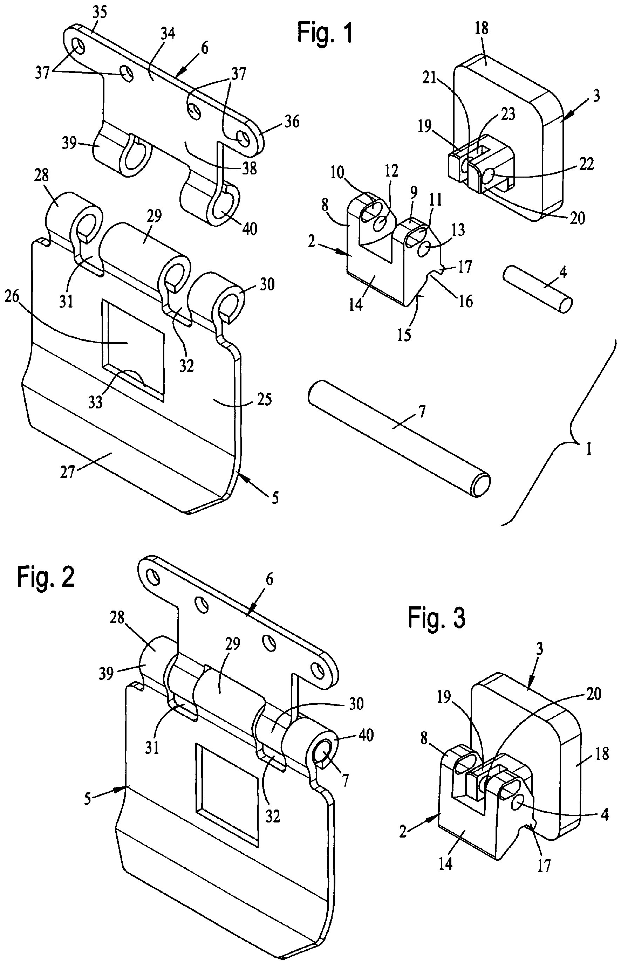

Fig. 1 zeigt eine perspektivische Explosionsdarstellung einer möglichen Ausführungsvariante der Bestandteile eines erfindungsgemäßen Verschlusses 1. Dieser Verschluss 1 besteht aus einem Spannhebel 2, einem zugehörigen Lagerbock 3, an welchem der Spannhebel 2 im Betrieb über eine Schwenkachse 4 schwenkbar gelagert ist. Des Weiteren ist eine Verschlussklappe 5 vorgesehen, welche im Einsatz an einem Lagerelement 6 über eine Drehachse 7 drehbar gelagert ist.

Der Spannhebel 2 ist bei der dargestellten Ausführungsvariante blockartig ausgebildet und weist zwei vertikal nach oben gerichtete Lagerschenkel 8 und 9 auf. In ihrem oberen Endbereich ist jeder der Lagerschenkel 8, 9 mit einem Langloch 10 bzw. 11 versehen, welche im Betrieb zur Lagesicherung des Spannhebels 2 in seiner geschlossenen Spannstellung dienen. Unterhalb dieser Langlöcher 10 und 11 sind in den beiden Lagerschenkeln 8 und 9 Lagerbohrungen 12 und 13 vorgesehen, über welche der Spannhebel 2 am Lagerbock 3 schwenkbar aufnehmbar ist.

Des Weiteren ist aus Fig. 1 ersichtlich, dass der Spannhebel 2 unterhalb seiner Lagerbohrungen 12 und 13 bzw. unterhalb seiner beiden Lagerschenkel 8 und 9 einen Spannblock 14 bildet, welcher rückseitig zum Lagerbock 3 hin eine schräg verlaufende Eingriffsfläche 15 bildet. Unterhalb der beiden Lagerbohrungen 12 und 13 mündet diese Einlauffläche 15 in eine nutartige Vertiefung 16, welche rückseitig, zum Lagerbock 3 hin, durch einen nach unten und schräg nach hinten, etwa radial zu den beiden Lagerbohrungen 12 und 13 verlaufend, vorstehenden Anschlagsteg 17 begrenzt ist.

Der Lagerbock 3 ist bei der dargestellten Ausführungsvariante plattenartig ausgebildet und weist dementsprechend eine Montageplatte 18 auf, welche vorderseitig mit zwei zum Spannhebel 2 hin vorstehende Lagerzungen 19 und 20 versehen ist. Jede dieser Lagerzungen 19 bzw. 20 ist mit einer Durchgangsbohrung 21 bzw. 22 versehen, welche zur schwenkbaren Lagerung des Spannhebels 2 über die Schwenkachse 4 dienen. Die beiden Lagerzungen 19 und 20 sind zwischen die beiden Lagerschenkel 8 und 9 des Spannhebels 2 passend einschiebbar, so dass der Spannhebel 2 im montierten Zustand mit geringem Spiel schwenkbar von den Lagerzungen 19 und 20 aufgenommen wird. Die beiden Lagerzungen 19 und 20 bilden zwischen sich im Bereich der Durchgangsbohrungen 21 und 22 eine Ausfräsung 23, innerhalb welcher zur Fixierung der geöffneten Schwenkstellung des Spannhebels 2 relativ zum Lagerbock 3 eine Schenkelfeder einsetzbar ist (in der Zeichnung nicht dargestellt) .

Wie aus Fig. 1 ersichtlich ist, ist die Verschlussklappe 5 bei dem dargestellten Ausführungsbeispiel plattenartig ausgebildet und weist einen ebenen Riegelabschnitt 25 auf, welcher mit einem rechteckförmigen Durchbruch 26 versehen ist. Unterhalb dieses Durchbruches 26 ist am Riegelabschnitt 25 eine nach vorne, außen angebogene Betätigungsleiste 27 angeformt, welche im Einsatz manuell hintergreifbar ist, so dass die Verschlussklappe 5 über diese Betätigungsleiste 27 äußerst einfach insbesondere geöffnet werden kann. Oberhalb des Riegelabschnittes 25 sind an diesem drei Lageraugen 28, 29 und 30 angeformt. Zwischen den beiden äußeren Lageraugen 28 und 30 und dem mittleren Lagerauge 29 ist jeweils ein Aufnahmebereich 31 bzw. 32 gebildet. Über diese Lageraugen 28, 29 und 30 ist die Verschlussklappe 5 am Lagerelement 6 über die Drehachse 7 im Betrieb drehbar gelagert.

Der rechteckige Durchbruch 26 ist in seiner Dimensionierung derart ausgebildet, dass der Spannhebel 2 mit seinem Spannblock 14 in der geöffneten Schwenkstellung des Spannhebels 2 durch den Durchbruch 26 hindurch steckbar ist. Dieser Durchbruch 26 bildet mit seiner Unterkante eine Eingriffsleiste 33, welche im Betrieb, insbesondere im geschlossenen Zustand des Verschlusses 1, mit der nutartigen Vertiefung 16 des Spannhebels 2 formschlüssig in Eingriff bringbar ist.

Das Lagerelement 6 weist in seinem oberen Endbereich eine Art Lagersteg 34 auf, welcher zwei seitlich vorstehende Montagezungen 35 und 36 bildet. Dieser Lagersteg 34 ist beim vorliegenden Ausführungsbeispiel mit insgesamt vier Montagebohrungen 37 versehen, über welche das Lagerelement 6 beispielsweise im Randbereich eines Behälterdeckels feststehend montierbar ist. Unterhalb des Lagersteges 34 bildet das Lagerelement 6 einen Verbindungsabschnitt 38, an welchem zwei nach unten, zur Verschlussklappe 5 hin vorstehende Lageraugen 39, 40 angeformt sind. Der Abstand dieser Lageraugen 39 und 40 sowie deren Breite ist dabei derart gewählt, dass diese Lageraugen 39 und 40 mit geringem Spiel in die beiden Aufnahmebereiche 31 und 32 der drei Lageraugen 28, 29 und 30 der Verschlussklappe 5 einsetzbar sind. In diesem montierten Zustand ist somit die Verschlussklappe 5 über die Drehachse 7 drehbar mit dem Lagerelement 6 koppelbar.

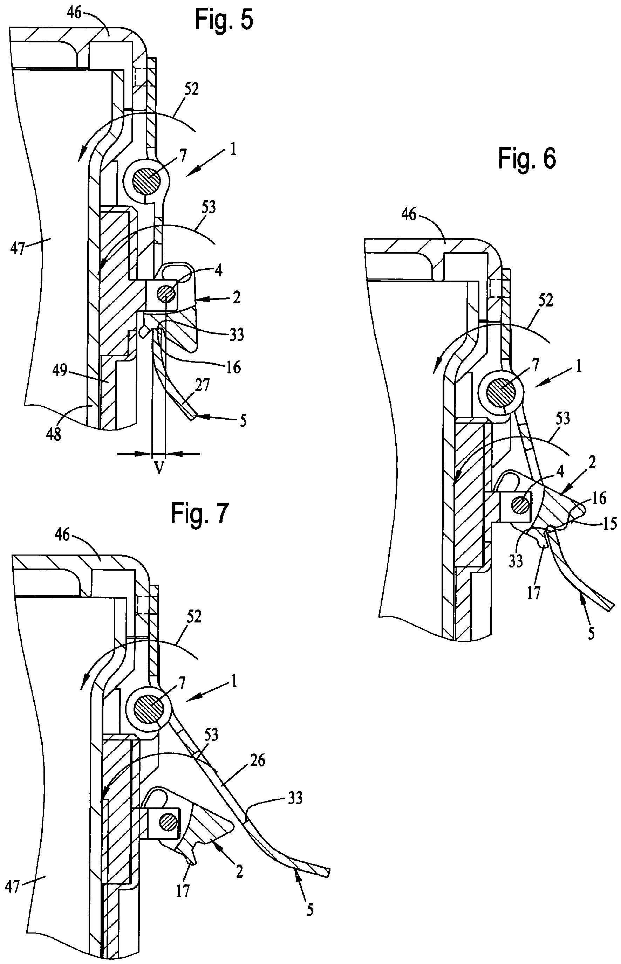

Diesen montierten Zustand zeigt Fig. 2 in perspektivischer Darstellung. Es ist erkennbar, dass die beiden Lageraugen 39 und 40 des Lagerelementes 6 in die beiden Aufnahmebereiche 31 und 32 zwischen den Lageraugen 28 und 29 bzw. 29 und 30 der Verschlussklappe 5 eingesetzt sind. Die Drehachse 7 ist durch alle Lageraugen 28, 29, 30 und 39, 40 hindurch gesteckt. Zur Sicherung dieser Verbindungsposition können die Lageraugen 28, 29, 30 und/oder 39, 40 unter radialer Vorspannung stehen, so dass die Drehachse 7 klemmend in den Lageraugen 28 bis 30 und 29, 40 gehalten ist. In der in Fig. 6 dargestellten relativen Drehstellung befindet sich die Verschlussklappe 5 etwa in ihrer geschlossenen Schließstellung.

Fig. 3 zeigt eine perspektivische Darstellung des am Lagerbock 3 montierten Spannhebels 2. Die beiden Lagerzungen 19 und 20 des Lagerbockes 3 sind zwischen die beiden Lagerschenkel 8 und 9 des Spannhebels 2 eingesetzt, so dass der Spannhebel 2 über die Schwenkachse 4 schwenkbar am Lagerbock 3 aufgenommen ist. Fig. 3 zeigt ebenfalls eine relative Schwenkstellung des Spannhebels 2 zum Lagerbock 3, in welcher sich der Spannhebel 2 etwa in seiner geschlossenen Spannstellung befindet. In dieser Spannstellung liegt der Spannhebel 2 mit seinem nach unten und zur Montageplatte 18 hin vorstehenden Anschlagsteg 17 außenseitig auf der Montageplatte 18 an. Somit bildet dieser Anschlagsteg 17 gleichzeitig einen definierten Anschlag zur Festlegung der "geschlossenen" Spannstellung des Spannhebels 2.

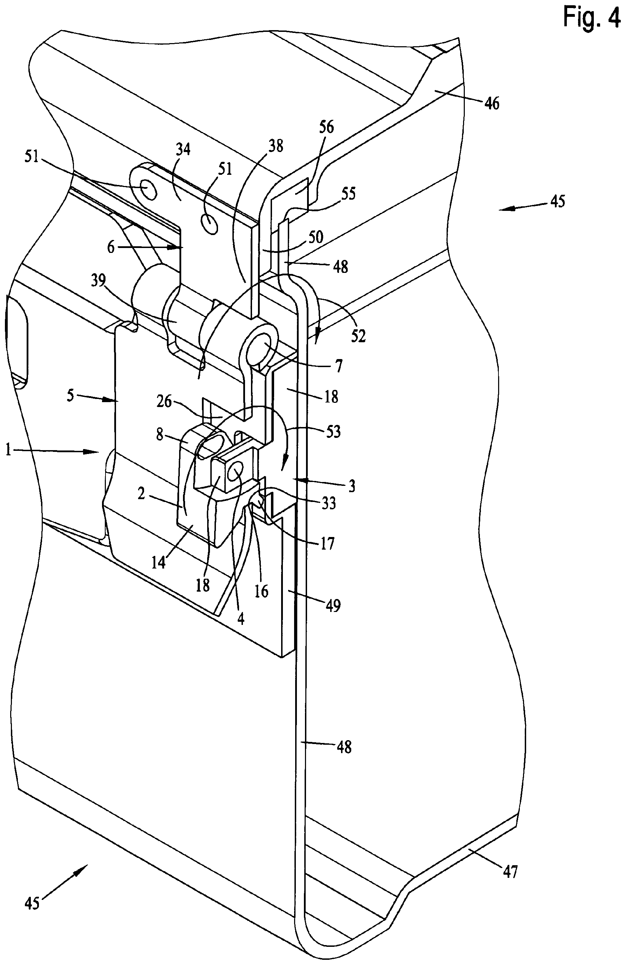

Fig. 4 zeigt eine teilweise perspektivische Schnittdarstellung eines Sterilcontainers 45, welcher einen oberen Behälterdeckel 46 sowie ein kastenartiges Unterteil 47 aufweist. In Fig. 4 ist beispielhaft die vordere Begrenzungswand 48 des Unterteils 47 in perspektivischem Teilschnitt erkennbar. An dieser Begrenzungswand 48 ist der Lagerbock 3 feststehend montiert. In diesem, in Fig. 4 dargestellten perspektivischen Teilschnitt, ist von diesem Lagerbock 3 einerseits die Montageplatte 18 erkennbar, mit welcher der Lagerbock 3 flächig an der vorderen Begrenzungswand 48 anliegt. Dabei kann der Lagerbock 3 rückseitig mit der Begrenzungswand 48 verschraubt sein oder über ein separates, mehrfach abgebogenes Halteblech 49 an der Begrenzungswand 48 befestigt sein. Beim vorliegenden Ausführungsbeispiel ist der Lagerbock 3 nicht mit der Begrenzungswand 48 verschraubt, so dass die Seitenwand 48 nicht mit Durchgangsbohrungen versehen werden muss und somit nicht die Gefahr des "Undichtwerdens" besteht. Das "Halteblech" 49 kann auch als Frästeil aus Aluminium ausgebildet und mit der Seitenwand 48 nach einem speziellen Verfahren verpresst sein.

In Fig. 4 ist des Weiteren die nach außen vorstehende Lagerzunge 19 sowie die montierte Schwenkachse 4 erkennbar. Auf dieser Schwenkachse 4 ist der Spannhebel 2 mit seinem Lagerschenkel 8 schwenkbar gelagert und befindet sich in Fig. 4 in seiner geschlossenen Spannstellung. Es ist erkennbar, dass bei dieser Ausführungsvariante der Anschlagsteg 17 außenseitig auf einem Teil des Montagebleches 49 anliegt.

An einer vertikal nach unten stehenden Stegwand 50 des Behälterdeckels 46 ist das Lagerelement 6 mit seinem Lagersteg 34 feststehend angeordnet. Zur feststehenden Verbindung können hier Schrauben, oder wie dargestellt, Befestigungsniete 51 vorgesehen sein. Das Lagerelement 6 ist entsprechend der vertikalen Ausrichtung der Stegwand 50 mit einem Verbindungsabschnitt 38 ebenfalls vertikal ausgerichtet und befindet sich mit seinem Lagerauge 39 im oberen Endbereich vor der vorderen Begrenzungswand 48.

Die vertikal nach unten gerichtete Verschlussklappe 5 steht mit dem Spannhebel 2 formschlüssig in Eingriff. Es ist erkennbar, dass die Eingriffsleiste 33 des Durchbruches 26 mit der Vertiefung 16 des Spannhebels 2 bzw. dessen Spannblock 14 formschlüssig in Eingriff steht. Dabei befinden sich in dieser geschlossenen Stellung des Verschlusses 1 die Vertiefung 16 und die Eingriffsleiste 33 relativ zur Schwenkachse 4 versetzt zur Begrenzungswand 48 hin, so dass sich in dieser geschlossenen Stellung des Verschlusses 1 dieser Eingriffsbereich zwischen dem Spannhebel 2 und der Verschlussklappe 5 über dem Totpunkt des Spannhebels 2 befindet. Durch diese Ausgestaltung wird der Spannhebel 2 entsprechend in seiner in Fig. 4 dargestellten Spannstellung selbständig gehalten.

Wird nun die Verschlussklappe 5 um die Drehachse 7 in Richtung des Pfeiles 52 gedreht, so wird ebenfalls eine Schwenkbewegung des Spannhebels 2 in Richtung des Pfeiles 53 um dessen Schwenkachse 4 bewirkt. Dies aufgrund des formschlüssigen Eingriffes der Eingriffsleiste 33 der Verschlussklappe 5 mit der Vertiefung 16 des Spannblockes 14. Damit wird der Spannhebel 2 beim Öffnen der Verschlussklappe 5 zwangsläufig aus der in Fig. 4 dargestellten Spannstellung in eine geöffnete Position über den Totpunkt hinaus in eine geöffnete Schwenkstellung gebracht.

Hierzu zeigen die Fig. 5, 6 und 7 den entsprechend zugehörigen Bewegungsablauf. Hierzu sei noch angemerkt, dass zwischen der Oberkante 55 und dem Behälterdeckel 46 ein Dichtelement 56 angeordnet sein kann, welches in diesem geschlossenen, verspannten Zustand des Behälterdeckels 46, mit dem Unterteil 47 unter elastischer Vorspannung steht.

Fig. 5 zeigt eine teilweise Schnittdarstellung des Behälterdeckels 46 in seinem auf dem Unterteil 47 montierten Zustand mit dem montierten Verschluss 1 in seiner geschlossenen Stellung, wie diese bereits zu Fig. 4 beschrieben wurde.

Aus Fig. 5 ist insbesondere erkennbar, dass sich in dieser geschlossenen Stellung die Eingriffsleiste 33 zusammen mit der Vertiefung 16 um einen Versatz **V** relativ zur Schwenkachse 4 versetzt zur Begrenzungswand 48 hin befinden. Aufgrund dieser "Übertotpunkt-Anordnung" wird aufgrund der bestehenden beispielsweise durch das elastische Dichtelement 56 bewirkten Zugkräfte, welche am Verschluss in dieser geschlossenen Stellung wirken, ein Drehmoment entgegen der Richtung des Pfeiles 53 bewirkt, so dass der Spannhebel 2 unter Vorspannung in der dargestellten Spannstellung gehalten wird. Aufgrund des formschlüssigen Eingriffes der Eingriffsleiste 33 mit der Vertiefung 16 wird dementsprechend gleichzeitig auch die Verschlussklappe 5 in ihrer dargestellten Schließstellung gehalten. Der "hintere" Anschlagsteg 17 liegt bei dieser dargestellten Ausführungsvariante an einem Teil des Montagebleches 49 außenseitig an, so dass diese Spannstellung des Spannhebels 2 bzw. die Schließstellung der Verschlussklappe 5 präzise definiert ist. Zum Öffnen des Verschlusses 1 kann nunmehr durch Betätigen der Verschlussklappe 5 im Bereich ihrer Betätigungsleiste 27 in Richtung des Pfeiles 52 ein Öffnen des Verschlusses 1 bewirkt werden.

Hierzu zeigt Fig. 6 eine Zwischenstellung, bei welcher die Verschlussklappe 5 in Richtung des Pfeiles 52 soweit geöffnet wurde, bis der Spannhebel 2 in Richtung des Pfeils 53 in seine geöffnete Schwenkstellung gelangt ist. In dieser Schwenkstellung kann der Spannhebel 2 beispielsweise durch eine im Bereich der Schwenkachse 4 angeordnete Schenkelfeder sicher gehalten sein. Diese geöffnete Schwenkstellung des Spannhebels 2 kann dabei durch eine spezielle Formgebung des Spannhebels 2 fixiert sein, so dass dieser nicht weiter in Richtung des Pfeiles 53 bewegt werden kann.

Weiter ist aus Fig. 6 andeutungsweise erkennbar, dass nunmehr die Eingriffsleiste 33 etwa im "Umfangsbereich" der Eingriffsfläche 15 liegt. Damit steht die Eingriffsleiste 33 mit der Vertiefung 16 des Spannhebels 2 nicht mehr in Eingriff. Durch weiteres Drehen der Verschlussklappe 5 in Richtung des Pfeiles 52 gelangt die Verschlussklappe 5 somit mit ihrem Durchbruch 26 bzw. der Eingriffsleiste 33 vollständig mit dem Spannhebel 2 außer Eingriff, wie dies aus Fig. 7 ersichtlich ist. In dieser geöffneten Stellung kann der Behälterdeckel 46 vom Unterteil 47 abgehoben werden.

Bei entsprechender Schließbewegung entgegen des Pfeiles 52, aus der in Fig. 7 dargestellten Schwenklage der Verschlussklappe 5, gelangt der Riegelabschnitt 25 mit seiner Eingriffsleiste 33 mit dem Anschlagsteg 17 des Spannhebels 2 wiederum formschlüssig in Eingriff, sobald sich die Verschlussklappe 5 in der in Fig. 6 dargestellten Position befindet. Durch weiteres Drehen der Verschlussklappe 5 entgegen des Pfeiles 52 wird somit der Spannhebel 2 über den Anschlagsteg 17 entgegen des Pfeiles 53 in seine geschlossene Spannstellung aus Fig. 5 zurück bewegt.

Insbesondere aus den Fig. 5 bis 7 ist erkennbar, dass der erfindungsgemäße Verschluss 1 in einer äußerst einfachen und sicheren Weise betätigbar ist. Es müssen keinerlei Bauteile insbesondere vor dem Schließvorgang manuell eingehängt werden. Auch ein Aushängen zweier oder mehrerer Funktionsbauteile beim Öffnen ist nicht notwendig. Durch die erfindungsgemäße Ausgestaltung ist somit eine äußerst einfache Handhabung des Verschlusses 1 gewährleistet.

Des Weiteren sind aufgrund der speziellen Ausgestaltung auch äußerst große Spannkräfte zum Verschließen des Behälterdeckels 46 am Unterteil 47 bewirkbar. Insbesondere ist in der geschlossenen Betriebsstellung des Verschlusses 1 ein Abheben des Behälterdeckels 46 vom Unterteil 47 und somit ein "Undichtwerden" des Sterilcontainers sicher ausgeschlossen.

Da die Eingriffsleiste 33 nur eine relativ kleine "drehende" Relativbewegung zur Vertiefung 16 beim Öffnen und beim Schließen ausführt, wird auch relativ geringer Verschließ erreicht.

## Patentansprüche

1. Verschluss (1) zum Verriegeln eines aus einem Behälterdeckel (46) und einem kastenartigen Unterteil (47) bestehenden Sterilcontainers (45), wobei der Behälterdeckel (46) und das Unterteil (47) zur Bildung eines geschlossenen Sterilraums abnehmbar und mittels umlaufend zwischen dem Behälterdeckel (46) und Unterteil (47) angeordneten Dichtungsmitteln (56) luftdicht auf das Unterteil (47) aufsetzbar und mit diesem verbindbar ist, wobei der Verschluss (1) einen Spannhebel (2) aufweist, welcher aus einer geschlossenen über dem Todpunkt liegenden Spannstellung um eine Schwenkachse (4) schwenkbar in eine geöffnete Schwenkstellung bringbar ist und,
dass eine um eine Drehachse (7) drehbare Verschlussklappe (5) vorgesehen ist, welche in ihrer Schließstellung mit dem Spannhebel (2) formschlüssig in Zugverbindung steht,
**dadurch gekennzeichnet,**
**dass** der Spannhebel (2) über die formschlüssige Verbindung mit der Verschlussklappe (5) während der Drehbewegung (52) der Verschlussklappe (5) aus ihrer Schließstellung in ihre geöffnete Drehstellung durch die Verschlussklappe (5) in seine geöffnete Schwenkstellung bringbar ist und,
**dass** die Verschlussklappe (5) nach einer weiteren Drehbewegung (52) in ihre vollständig geöffneten Endstellung mit dem Spannhebel (2) außer Eingriff gelangt und,
**dass** der Spannhebel (2) in seiner geöffneten Schwenkstellung fixiert ist und,
**dass** die Verschlussklappe (5) beim Schließvorgang mit dem Spannhebel (2) formschlüssig in Eingriff gelangt und der Spannhebel (2) durch die Verschlussklappe (5) in seine geschlossene Spannstellung verstellbar ist.

2. Verschluss nach Anspruch 1, **dadurch gekennzeichnet, dass** der Spannhebel (2) blockartig ausgebildet ist und zwei voneinander beabstandete Lagerschenkel (8, 9) aufweist, welche jeweils mit einer Lagerbohrung (12, 13) versehen sind, über welche der Spannhebel (2) an einem am Sterilcontainer (45) befestigten Lagerbock (3) schwenkbar gelagert ist.

3. Verschluss nach Anspruch 2, **dadurch gekennzeichnet, dass** der Spannhebel (2) ausgehend von den Lagerschenkeln (8, 9) einen Spannblock (14) bildet und,
dass der Spannblock (2) in seinem den Lagerschenkeln (8, 9) gegenüber liegenden Endbereich eine parallel zu seiner Schwenkachse (4) verlaufende Vertiefung (16) aufweist, mit welcher die Verschlussklappe (5) in formschlüssig und beweglich in Eingriff bringbar ist.

4. Verschluss nach Anspruch 3, **dadurch gekennzeichnet, dass** die Vertiefung (16) "rückseitig" zum Sterilcontainer (45) hin durch einen etwa radial zur Schwenkachse (4) verlaufenden Anschlagsteg (17) begrenzt ist, welcher mit der Verschlussklappe (5) beim Schließvorgang in Wirkverbindung gelangt und, dass sich an die Vertiefung (26) dem Anschlagsteg (17) gegenüberliegend eine Eingriffsfläche (15) anschließt.

5. Verschluss nach Anspruch 1, **dadurch gekennzeichnet, dass** die Verschlussklappe (5) über ein Lagerelement (6) drehbar am Sterilcontainer (45) gelagert ist und,
dass die Verschlussklappe (5) einen zentralen Durchbruch (26) mit einer parallel zur Drehachse (7) verlaufenden Eingriffsleiste aufweist, mit welcher die Verschlussklappe (5) mit dem Spannhebel (2) formschlüssig in Eingriff bringbar ist.

6. Verschluss nach Anspruch 3 und 5, **dadurch gekennzeichnet, dass** der Spannhebel (2) mit seinem Spannblock (14) im geschlossenen Zustand des Verschlusses (1) den Durchbruch (26) der Verschlussklappe (5) durchragt und,
dass der Durchbruch (26) eine Eingriffsleiste (33) bildet, welche im geschlossenen Zustand des Verschlusses (1) mit der Vertiefung (16) des Spannblocks (14) formschlüssig und relativ zu diesem drehbar in Eingriff steht.

## Claims

1. Closure (1) for locking a sterile container (45) consisting of a container lid (46) and a box-like lower part (47), wherein, in order to form a closed sterile chamber, the container lid (46) and the lower part (47) can be removably placed on and connected to the lower part (47) in an air-tight manner by means of sealing means (56) arranged peripherally between the container lid (46) and lower part (47), wherein the closure (1) has a clamping lever (2) which can be brought from a closed clamping position situated above the dead centre into an opened pivoted position by being pivotable about a pivot pin (4), and that there is provided a closure flap (5) which can be rotated about a rotation pin (7) and which, in its closed position, is positively in tensile connection with the clamping lever (2),
**characterized**
**in that**, during the rotary movement (52) of the closure flap (5) from its closed position into its opened rotated position, the clamping lever (2) can be brought, via the positive connection to the closure flap (5), into its opened pivoted position by means of the closure flap (5), and
**in that**, after a further rotary movement (52) into its completely opened end position, the closure flap (5) is brought out of engagement with the clamping lever (2), and
**in that** the clamping lever (2) is fixed in its opened pivoted position, and
**in that**, during the closing operation, the closure flap (5) is brought positively into engagement with the clamping lever (2), and the clamping lever (2) can be moved into its closed clamping position by means of the closure flap (5).

2. Closure according to Claim 1, **characterized in that** the clamping lever (2) is of block-like design and has two bearing legs (8, 9) which are spaced apart from one another and which are each provided with a bearing hole (12, 13) via which the clamping lever (2) is pivotably mounted on a bearing support (3) fastened to the sterile container (45).

3. Closure according to Claim 2, **characterized in that** the clamping lever (2) forms a clamping block (14) starting from the bearing legs (8, 9), and
**in that**, in its end region situated opposite to the bearing legs (8, 9), the clamping block (2) has a depression (16) which extends parallel to its pivot pin (4) and by means of which the closure flap (5) can be brought positively and movably into engagement.

4. Closure according to Claim 3, **characterized in that** the depression (16) is delimited "on the rear side" towards the sterile container (45) by a stop web (17) which extends approximately radially to the pivot pin (4) and which comes into operative connection with the closure flap (5) during the closing operation, and
**in that** an engagement surface (15) adjoins the depression (26) opposite to the stop web (17).

5. Closure according to Claim 1, **characterized in that** the closure flap (5) is rotatably mounted on the sterile container (45) via a bearing element (6), and
**in that** the closure flap (5) has a central aperture (26) with an engagement strip which extends parallel to the rotation pin (7) and with which the closure flap (5) can be brought positively into engagement with the clamping lever (2) .

6. Closure according to Claims 3 and 5, **characterized in that**, in the closed state of the closure (1), the clamping lever (2) engages by way of its clamping block (14) through the aperture (26) in the closure flap (5), and
**in that** the aperture (26) forms an engagement strip (33) which, in the closed state of the closure (1), is positively in engagement with the depression (16) of the clamping block (14) so as to be rotatable relative to the latter.

## Revendications

1. Fermeture (1) pour le verrouillage d'un conteneur stérile (45) se composant d'un couvercle de récipient (46) et d'une partie inférieure en forme de caisse (47), dans laquelle le couvercle de récipient (46) et la partie inférieure (47) peut être enlevé et posé sur la partie inférieure (47) d'une façon étanche à l'air au moyen de moyens d'étanchéité (56) disposés en périphérie entre le couvercle de récipient (46) et la partie inférieure (47) et assemblé à celle-ci pour la formation d'une chambre stérile fermée, dans laquelle la fermeture (1) présente un levier de serrage (2), qui peut être amené d'une position de serrage fermée située au-delà du point mort par pivotement autour d'un axe de pivotement (4) dans une position pivotée ouverte, et qu'il est prévu un clapet de fermeture (5) pouvant tourner autour d'un axe de rotation (7), qui dans sa position de fermeture est en relation de traction par emboîtement avec le levier de serrage (2),
**caractérisée en ce que** le levier de serrage (2) peut être amené par l'assemblage par emboîtement avec le clapet de fermeture (5), pendant le mouvement de rotation (52) du clapet de fermeture (5) de sa position de fermeture à sa position rotative ouverte, par le clapet de fermeture (5) dans sa position pivotée ouverte, et **en ce que** le clapet de fermeture (5) quitte l'engagement avec le levier de serrage (2) après un autre mouvement de rotation (52) dans sa position finale entièrement ouverte, et **en ce que** le levier de serrage (2) est fixé dans sa position pivotée ouverte, et **en ce que** le clapet de fermeture (5) vient en engagement par emboîtement avec le levier de serrage (2) lors de l'opération de fermeture et le levier de serrage (2) peut être déplacé par le clapet de fermeture (5) dans sa position de serrage fermée.

2. Fermeture selon la revendication 1, **caractérisée en ce que** le levier de serrage (2) est réalisé en bloc et présente deux branches de palier (8, 9) espacées l'une de l'autre, qui sont chacune dotées d'un alésage de palier (12, 13), par lequel le levier de serrage (2) est monté de façon pivotante sur un support de palier (3) fixé au conteneur stérile (45).

3. Fermeture selon la revendication 2, **caractérisée en ce que** le levier de serrage (2) forme à partir des branches de palier (8, 9) un bloc de serrage (14), et **en ce que** le bloc de serrage (2) présente dans sa région d'extrémité opposée aux branches de palier (8, 9) un creux (16) s'étendant parallèlement à son axe de pivotement (4), avec lequel le clapet de fermeture (5) peut être amené en engagement par emboîtement et de façon mobile.

4. Fermeture selon la revendication 3, **caractérisée en ce que** le creux (16) est limité "côté dorsal" vers le conteneur stérile (45) par une nervure de butée (17) s'étendant environ radialement à l'axe de pivotement (4), qui vient en liaison active avec le clapet de fermeture (5) lors de l'opération de fermeture, et **en ce qu'**une face d'engagement (15) se raccorde au creux (26) à l'opposé de la nervure de butée (17).

5. Fermeture selon la revendication 1, **caractérisée en ce que** le clapet de fermeture (5) est monté sur le conteneur stérile (45) de façon rotative au moyen d'un élément de palier (6), et **en ce que** le clapet de fermeture (5) présente un passage central (26) avec une baguette d'engagement s'étendant parallèlement à l'axe de rotation (7), avec laquelle le clapet de fermeture (5) peut être amené en engagement par emboîtement avec le levier de serrage (2).

6. Fermeture selon la revendication 3 et 5, **caractérisée en ce que** le levier de serrage (2) passe avec son bloc de serrage (14) dans l'état fermé de la fermeture (1) à travers le passage (26) du clapet de fermeture (5), et **en ce que** le passage (26) forme une baguette d'engagement (33), qui dans l'état fermé de la fermeture (1) est en engagement avec le creux (16) du bloc de serrage (14) par emboîtement et de façon rotative par rapport à celui-ci.
